# EUROPEAN PATENT SPECIFICATION

(11) **EP 1 453 428 B1**
(45) Date of publication and mention of the grant of the patent: **04.04.2007**
(21) Application number: 02783312.8
(22) Date of filing: 09.12.2002
(51) Int. Cl.: A61B 17/70

(54) **ARTICULATED VERTEBRAL FIXING**
GELENKIGE WIRBELFIXIERUNG
FIXATION VERTEBRALE ARTICULEE

(30) Priority: 09.12.2001 EG 20011313; 10.03.2002 EG 20020252; 10.03.2002 EG 20020253; 10.03.2002 EG 20020254
(43) Date of publication of application: 08.09.2004
(73) Proprietor: UNIVERSITY COLLEGE LONDON, London WC1E 6BT (GB)
(72) Inventor: BAYOUMI, Hazem Elsebaie, Giza (EG)
(74) Representative: Whitaker, Iain Mark
(86) International application number: PCT/GB2002/005564
(87) International publication number: WO 2003/053264

(56) References cited:
- WO-A-01/67973
- US-A- 5 628 740
- US-A- 5 649 926
- US-A- 5 735 851

## Description

The present invention is concerned with a vertebral fixing for use in a spinal fixation system. Vertebral fixings in the form of screws and hooks are used in spinal surgery to secure the elements of the spine to a support structure, such as a shaft, for the purpose of encouraging the spine to heal.

Conventional spinal fixings consist of a fixing part ordinarily provided by a screw or hook which can be fixed to the vertebrae. The projecting end of the screw or hook is in the form of a rigid threaded rod extension. A connector is provided for connecting the screw to a shaft. Commonly the connector consists of a collar which slips over the extension and is retained by a nut threaded on to the extension. The connector is also provided with a sleeve, or means to form a sleeve which envelopes and grips a part of the shaft. The shaft in turn will extend over some part of the length of the spine and be connected to several screws. In the course of treatment the nuts are tightened to provide support and/or corrective forces to the spine in order to promote healing.

US-A-5735851 discloses a vertebral fixing according to the preamble of claim 1.

It is a problem to connect the connectors to the rod because the screws must be located where the vertebrae are able to sustain the loads to be imposed on them and such sites are severely limited, particularly in view of the conditions being treated. In an effort to alleviate this problem connectors have been designed which have a degree of articulation. However, such connectors have been found to be unsatisfactory in that the forces applied when tightening the nut are misdirected and so inclined to pull the screw or hook from the vertebrae.

In an effort to alleviate the aforementioned problems the present invention provides a vertebral fixing according to claim 1.

Vertebral fixings constructed in accordance with the present invention will now be described, by way of example only, with reference to the accompanying figures, wherein:
Figure 1A is a side elevation of a first embodiment,
Figure 1 B is a side elevation of the first embodiment at 90 degrees, with a connector attached,
Figure 1C shows a connector,
Figure 2 is a perspective view of a second embodiment,
Figure 3A is a perspective view of a third embodiment, with the extension part omitted for clarity,
Figure 3B is a section on A-A through the third embodiment showing the extension,
Figure 4 is a perspective view of a fourth embodiment.

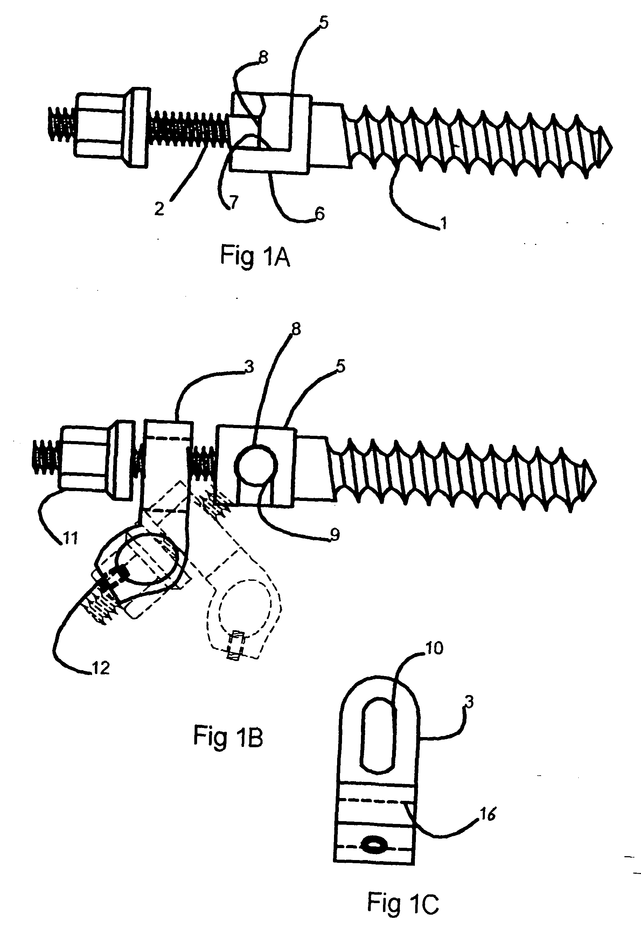

The vertebral fixing of the first embodiment, shown in figures 1A-C has a fixing part provided by a screw 1, an elongate extension provided by a rod 2 which is threaded to allow the position of a connector 3 to be accurately controlled on the rod by tightening a nut 11. The extension connects to the fixing part by means of an articulated coupling 5.

The articulated coupling 5 permits the extension 2 to pivot only about an axis perpendicular to the screw or extension axis. In practice any articulated coupling which provides this feature may be suitable. However, in the embodiment shown the coupling consists of a head part 6 formed integrally on the projecting end of the fastening, A slot 7 is formed extending down from the open top of the head along the axis and radially out to one side. The slot is sized to receive the rod 2 and its extension radially to the side of the head permits the rod 2 to pivot as indicated in ghost lines in figure 1a. The rod is provided with a pin 8 which sits in a passage 9 formed perpendicular to the long axis of the screw 1 and so provides the necessary articulation. In the case of the screw the vertebral fixing is intended to project from the spine in a direction common to the axis of the screw and so the rod is capable of pivoting about an axis perpendicular to the intended direction of projection.

Although not shown in the figures, means is provided to prevent the pin from being displaced out of the passage. Such means may include deforming part of the head after insertion of the pin or providing a small projection on the periphery of the passage through which the pin snap engages.

In use the screw 1 is fastened into the vertebrae and twisted until the rod 2 can be inclined towards a support structure, which will commonly be a shaft extending over a substantial part of the length of the spine. The connector 3 is provided with an elongate slot 10 which sleeves over the rod 2 and is secured by means of a nut 11. In an end of the connector remote from the slot is formed a bore 16 extending substantially perpendicular to the through axis of the slot. The shaft which provides the support structure can relatively easily be received into the bore 16 and secured by means of a grub screw 12. When the spinal fixation system has been assembled, commonly by the incorporation of several vertebral fixings each connected to a common shaft, corrective or supportive forces can be applied to the vertebrae of the spine by tightening the nut 11 of each fixing assembly. Because of the articulation of the fixing the forces applied are more correctly directed to support the vertebrae and correct any spinal deformation than is possible with conventional fixings.

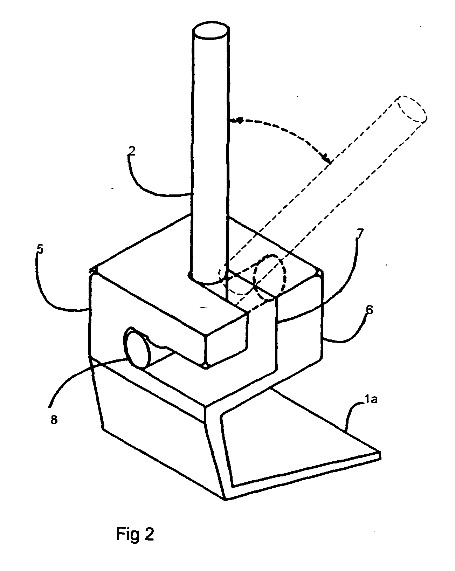

Figure 2 shows a second embodiment of the invention having an articulated coupling 5 in principle similar to that of the first embodiment, so that similar components are identified by similar numerals. However, the fastening part is provided by a hook 1a instead of a screw.

It may be noted that the slot 7 extends only partway throught the head 5 so that the head obstructs pivoting of the rod 2 to constrain it to pivot through an arc of only 90 degrees. The head may be adapted by the provision of projections or otherwise to constrain the range of pivoting of the rod 2.

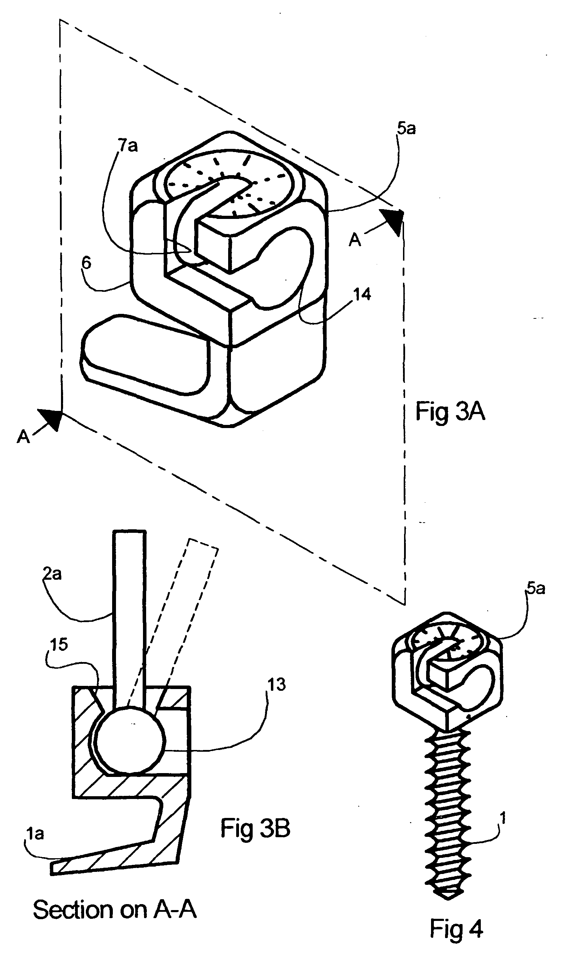

Figure 3 shows a third embodiment of the invention wherein the coupling 5 provides articulation about two axis. To achieve this the coupling 5a is formed as a ball joint with the ball 13 formed on the rod 2a. The ball end of the rod 2a is received into a socket 14 extending into the head 6 so that the rod 2a projects generally in the direction which the fastening is intended to project from the vertebrae through the slot 7a. However, the slot 7a is modified in that a funnel 15 is shaped into the open top of the slot 7a to act as a limiter and permits the rod 2a to articulate to a limited degree around two pivot axis through the ball 14.

Means is provided to prevent the ball 14 being displaced from the socket, for example, by deforming part of the head after assembly or by the provision of snap over projections on the periphery of the socket.

In use a connector such as the connector 3 or other conventional connectors are connected to the rod 2a in a manner similar to that described in detail in the first embodiment.

The fourth embodiment of the invention illustrated by figure 4 has a coupling identical to that of the second embodiment but the fastening part is provided by a screw 1.

It is likely that the different embodiments of the invention will be used together to form a vertebral fixing system because the different embodiments of the invention have particular advantages when used at particular locations. For example, hook fastenings are of particular use on the smaller vertebrae of the upper part of the spine, while screw fastenings are of advantage used at the lower larger vertebrae.

The head 5 of any embodiment may be given a shape to aid in the handling and insertion of the fixing part. In particular where the fixing part is a screw the head may have an external profile like that of a nut.

In the case of any of the embodiments the coupling, and hence the fixing part and the extension part, may be assembled after insertion of the fixing part.

## Claims

1. A vertebral fixing comprising a fixing part (1) and an elongate extension (2) extending from the fixing part (1) to which a connector (3) can be connected, said extension (2) being adapted so that the distance from the connector (3) to the fixing part (1) can be controlled, and an articulated coupling (5) whereby the extension part (2) is coupled to the fixing part (1) having an integral pivot pin (8) or ball (13) on the extension (2), whereby the articulated coupling comprises an integral head part (6) on the fixing part (1), whereby the extension (2) may be coupled to the head part (6) of the fixing part (1) solely by insertion of the pivot pin (8) or ball (13) of the extension (2) into the head part (6), **characterised in that** the head part (6) of the fixing part (1) has a slot (7) extending down through an open top of the head part (6) along the axis of the fixing part (1) initially, the slot (7) then extending outwardly to one side of the head part (6) whereby the extension (2) may be coupled to the head part (6) of the fixing part (1) by insertion of the pivot pin (8) or ball part (13) into the slot (7) from the side of the head part (6).

2. A vertebral fixing as claimed in Claim 1, wherein the fixing part (1) comprises a hook.

3. A vertebral fixing as claimed in Claim 1, wherein the fixing part (1) comprises a screw.

4. A vertebral fixing as claimed in any preceding claim, wherein the extension has a ball (13) to couple to the fixing part (1) and where the slot (7) extends down from the open top of the head part (6), the slot (7) is formed with a funnel-shape (15) to serve as a limiter and permit the extension (2) to articulate to a limited degree.

5. A vertebral fixing as claimed in any preceding claim, wherein integral retention means are provided on the head part (6) for retaining the pin (8) or ball (13) there within when installed.

6. A vertebral fixing as claimed in Claim 5, wherein the retention means comprises a small projection on the periphery of the slot (7).

7. A vertebral fixing as claimed in any preceding claim, wherein the head part (6) of the fixing part (1) is formed as a block with an external profile like that of a nut to facilitate handling and insertion of the fixing part (1).

8. A vertebral fixing according to any preceding claim, wherein the extension (2) has a ball (13) to couple to the fixing part (1).

9. A fixing according to any of Claims 1 to 7, wherein the extension (2) has a pivot pin (8) to couple to the fixing part (1).

## Patentansprüche

1. Wirbelfixierung, die einen Fixierungsteil (1) und eine längliche, sich von dem Fixierunzsteil (1) aus erstreckende, Erweiterung (2), mit der ein Verbindungsstück (3) verbunden werden kann, umfasst, wobei die Erweiterung (2) so eingerichtet ist, dass die Entfernung vom Verbindungsstück (3) zum Fixierungsteil (1) gesteuert werden kann, und eine Gelenkkupplung (5), durch die der Erweiterungsteil (2) an den Fixierungsteil (1) gekuppelt ist, einen integralen Drehzapfen (8) oder eine Kugel (13) an der Erweiterung (2) hat, wodurch, die Gelenkkupplung einen integralen Kopfteil (6) am Fixierungsteil (1) umfasst, wodurch die Erweiterung (2) nur durch Einsetzen des Drehzapfens (8) oder der Kugel (13) der Erweiterung (2) in den Kopfteil (6) an den Kopfteil (6) des Fixierungsteils (1) gekuppelt werden kann, **dadurch gekennzeichnet, dass** der Kopfteil (6) des Fixierungsteils (1) einen Schlitz (7) hat, der sich anfangs nach unten durch einen offenen Oberteil des Kopfteils (6) längs der Achse des Fixierungsteils (1) erstreckt, wobei sich der Schlitz (7) dann zu einer Seite des Kopfteils (6) nach außen erstreckt, wodurch die Erweiterung (2) durch Einsetzen des Drehzapfens (8) oder des Kugelteils (13) in den Schlitz (7) von der Seite des Kopfteils (6) an den Kopfteil (6) des Fixierungsteils (1) gekuppelt werden kann.

2. Wirbelfixierung nach Anspruch 1, wobei der lrixzerun,gsteil (1) einen Haken umfasst.

3. Wirbelfixierung nach Anspruch 1; wobei der Fixierungsteil (1) eine Schraube umfasst.

4. Wirbelfixierung nach einem der vorhergehenden Ansprüche, wobei die Erweiterung eine Kugel (13) zum Kuppeln an den Fixierungsteil (1) hat und wobei sich der Schlitz (7) nach unten vom offenen Oberteil des Kopfteils (6) erstreckt, wobei der Schlitz (7) mit einer Trichtergestalt (15) geformt ist, um als Begrenzer zu dienen und zu ermöglichen, dass die Erweiterung (2) bis zu einem begrenzten Grad gelenkig gelagert ist.

5. Wirbelfixierung nach einem der vorhergehenden Ansprüche, wobei integrale Festhaltemittel am Kopfteil (6) bereitgestellt werden, um den Zapfen (8) oder die Kugel (13) darin festzuhalten, wenn sie eingebaut sind.

6. Wijrbelfixierung nach Anspruch 5, wobei die Festhaltemittel einen kleinen Vorsprung am Umfang des Schlitzes (7) umfassen.

7. Wirbelfixierung nach einem der vorhergehenden Ansprüche, wobei der Kopfteil (6) des Fixierungsteils (1) als ein Block mit einem äußeren Profil, wie dem einer Mutter, geformt ist, um das Handhaben und Einsetzen des Fixierungsteils (1) zu erleichtern.

8. Wirbelfixierung nach einem der vorhergehenden Ansprüche, wobei die Erweiterung (2) eine Kugel (13) zum Kuppeln an den Fixierungsteil (1) hat.

9. Wirbelfixierung nach einem der Ansprüche 1 bis 7, wobei die Erweiterung (2) einen Drehzapfen (8) zum Kuppeln an den Fixierungsteil (1) hat.

## Revendications

1. Fixation vertébrale comprenant une partie de fixation (1) et une extension allongée (2), s'étendant à partir de la partie de fixation (1) sur laquelle un connecteur (3) peut être connecté, ladite extension (2) étant configurée de sorte que la distance entre le connecteur (3) et la partie de fixation (1) peut être contrôlée, et un accouplement articulé (5) assurant l'accouplement de la partie d'extension (2) à la partie de fixation (1), comportant une goupille de pivotement (8) ou une bille (13) solidaire sur l'extension (2), l'accouplement articulé comprenant une partie de tête solidaire (6) sur la partie de fixation (1), l'extension (2) pouvant être accouplée à la partie de tête (6) de la partie de fixation (1) uniquement par l'intermédiaire de l'insertion de la goupille de pivotement (8) ou de la bille (13) de l'extension (2) dans la partie de tête (6), **caractérisée en ce que** la partie de tête (6) de la partie de fixation (1) comporte une fente (7) s'étendant vers le bas à travers une partie supérieure ouverte de la partie de tête (6), initialement le long de l'axe de la partie de fixation (1), la fente (7) s'étendant ensuite vers l'extérieur, vers un côté de la partie de tête (6), l'extension (2) pouvant ainsi être accouplée à la partie de tête (6) de la partie de fixation (1) par insertion de la goupille de pivotement (8) ou de la partie de bille (13) dans la fente (7), à partir du côté de la partie de tête (6).

2. Fixation vertébrale selon la revendication 1, dans laquelle la partie de fixation (1) comprend un crochet.

3. Fixation vertébrale selon la revendication 1, dans laquelle la partie de fixation (1) comprend une vis.

4. Fixation vertébrale selon l'une quelconque des revendications précédentes, dans laquelle l'extension comporte une bille (13) pour accoupler à la partie de fixation (1), la fente (7) s'étendant vers le bas à partir de la partie supérieure ouverte de la partie de tête (6), la fente (7) ayant une forme en entonnoir (15) pour servir de moyen de limitation et permettre l'articulation de l'extension (2) à un degré limité.

5. Fixation vertébrale selon l'une quelconque des revendications précédentes, dans laquelle des moyens de retenue solidaires sont agencés sur la partie de tête (6) pour retenir la goupille (8) ou la bille (13) dans celle-ci après leur installation.

6. Fixation vertébrale selon la revendication 5, dans laquelle le moyen de retenue comprend une petite saillie sur la périphérie de la fente (7).

7. Fixation vertébrale selon l'une quelconque des revendications précédentes, dans laquelle la partie de tête (6) de la partie de fixation (1) a la forme d'un bloc, avec un profil extérieur semblable à celui d'un écrou pour faciliter la manipulation et l'insertion de la partie de fixation (1).

8. Fixation vertébrale selon l'une quelconque des revendications précédentes, dans laquelle l'extension (2) comporte une bille (13) pour assurer l'accouplement à la partie de fixation (1).

9. Fixation selon l'une quelconque des revendications 1 à 7, dans laquelle l'extension (2) comporte une goupille de pivotement (8) pour assurer l'accouplement à la partie de fixation (1).
